(19) **Europäisches Patentamt**

**European Patent Office**

**Office européen des brevets**

(11) **EP 0 621 034 B1**

(12) **FASCICULE DE BREVET EUROPEEN**

(45) Date de publication et mention
de la délivrance du brevet:
**02.07.2003   Bulletin 2003/27**

(51) Int Cl.⁷: **A61K 31/195**, A61K 7/40,
A61K 7/48, A61K 33/06,
A61P 17/02

(21) Numéro de dépôt: **94400840.8**

(22) Date de dépôt: **18.04.1994**

(54) **Composition contenant des complexes de l'aluminium pour le traitement des brûlures thermiques ou chimiques**

Aluminium Komplexe enthaltende Zusammensetzung zur Behandlung von chemischen oder thermischen Verletzungen

Composition containing aluminium complexes for the treatment of chemical or heat burns

(84) Etats contractants désignés:
**BE DE ES GB IT**

(30) Priorité: **19.04.1993   FR 9304585
06.07.1993   FR 9308278**

(43) Date de publication de la demande:
**26.10.1994   Bulletin 1994/43**

(73) Titulaire: **Blomet, épouse Meyer, Marie-Claude
F-75015 Paris (FR)**

(72) Inventeurs:
• **Blomet, Joel
95760 Valmondois (FR)**
• **Blomet, Marie-Claude épouse Meyer
75015 Paris (FR)**
• **Friard, Dominique épouse Jahan
78780 Maurecourt (FR)**

(74) Mandataire: **Boulinguiez, Didier et al
Cabinet Plasseraud
84, rue d'Amsterdam
75440 Paris Cedex 09 (FR)**

(56) Documents cités:
**FR-A- 2 604 900**

• **DATABASE WPI Week 9139, Derwent
Publications Ltd., London, GB; AN 91-284799 &
JP-A-3 188 149 (OTSUKA SEIYAKU KOJY) 16
Août 1991**

Remarques:
Le dossier contient des informations techniques présentées postérieurement au dépôt de la demande et ne figurant pas dans le présent fascicule.

## Description

L'invention a pour objet une composition physiologique pour le traitement des brûlures thermiques ou chimiques.

Elle vise également le procédé de préparation de cette composition, un concentré utile pour sa préparation ainsi que l'application de ladite composition pour le traitement des brûlures internes ou externes.

Lors de la manipulation de produits corrosifs et irritants, comme par exemple les acides forts ou les bases fortes, notamment dans les usines et les laboratoires, il est fréquent que des projections accidentelles de ces produits entrent en contact avec la peau, les cheveux ou les yeux. Il peut même arriver que ces produits soient ingérés de façon accidentelle.

De même, il est malheureusement fréquent que des produits chimiques réactifs, corrosifs ou irritants, utilisés par exemple pour le bricolage ou le jardinage, soient la cause de graves accidents domestiques, ces accidents étant d'autant plus regrettables et désolants qu'ils touchent le plus souvent de jeunes enfants. Parmi les produits en question, on peut citer par exemple les acides forts et les bases fortes, les décapants, les liquides et poudres de nettoyage, les agents détersifs, les solvants, les engrais, les insecticides, les fongicides et les désherbants. Ces produits peuvent entrer en contact accidentel avec une partie extérieure du corps, ou être ingérés.

Il est extrêmement important, lorsque ces accidents se produisent, de pouvoir agir rapidement pour empêcher, ou tout au moins limiter autant que faire se peut, l'apparition de brûlures aux conséquences irréversibles.

Il est évidemment d'usage courant de rincer la partie du corps atteinte avec de l'eau sans additif, par exemple l'eau du robinet. Toutefois, cette méthode simple, facilement applicable et accessible, et qui peut donner des résultats relativement satisfaisants sur des parties du corps assez peu fragiles telles que les mains, s'est révélée la plupart du temps insuffisante lorsqu'il s'est agi de traiter l'oeil, et il est évident que cette méthode est totalement inefficace lorsqu'il y a eu ingestion de produits corrosifs.

Des études ont montré que la neutralisation d'un acide fort ou d'une base forte par l'eau nécessite plusieurs heures, ce qui fait que dans le cas d'une atteinte de l'oeil par exemple, l'agent corrosif ou irritant poursuit son agression des cellules endothéliales, ce qui peut conduire à des effets catastrophiques et irréversibles.

De plus, il faut également souligner que les effets de cette lenteur d'action sont aggravés du fait que l'eau présente, par rapport à la peau ou à l'oeil notamment, une hypotonicité. Ainsi, dans le cas de l'oeil, l'eau pénètre par effet osmotique dans le stroma en entraînant avec elle une partie de l'agent agressif qui y provoque de l'oedème et des lésions dans les cellules endothéliales.

Afin de remédier à ces effets néfastes, on a proposé de "laver" les agents agressifs en cause par des solutions contenant un produit réactif d'effet contraire. Ainsi, on a proposé de laver les acides par une solution d'un composé à caractère de base faible et les bases par une solution d'un composé à caractère d'acide faible, ces solutions étant de préférence isotoniques avec la partie du corps à traiter, par exemple le liquide lacrymal dans le cas de l'oeil.

Il existe ainsi dans le commerce de telles solutions, par exemple à base d'acide borique pour laver les brûlures par les bases et à base de bicarbonate de sodium pour laver les brûlures par les acides.

De telles solutions donnent en général des résultats satisfaisants lorsque l'agent à l'origine de la brûlure est connu. Malheureusement, tel n'est pas souvent le cas dans la pratique courante de l'industrie ou du laboratoire, et a fortiori dans le cas d'accidents domestiques.

Il est donc souhaitable de disposer d'une solution utilisable et efficace quel que soit le type d'agent agressif.

Dans ce but, on a déjà proposé des tampons, c'est-à-dire des solutions comportant deux formes ioniques dérivées d'un même ion tel que par exemple l'ion phosphate, se transformant l'une en l'autre par réaction acide-base. Il existe ainsi sur le marché une solution comportant deux ions dérivés de l'ion phosphate $PO_4^{3-}$, à savoir les ions $HPO_4^{2-}$ et $H_2PO_4^{-}$

En fait, étant donné que les solutions de ce type ne présentent pas totalement l'efficacité recherchée, en raison notamment de la répartition des constantes d'équilibre des réactions mises en jeu, d'autres solutions bien meilleures ont été recherchées, proposées et adoptées. Ainsi, le brevet français 86 11754 a proposé une solution physiologique unique présentant une efficacité satisfaisante et agissant rapidement tant vis-à-vis des acides que des bases en ayant recours, pour réaliser cette solution, à un ou plusieurs ampholyte(s) choisi(s) de telle sorte que le pK acide d'une part, et le pK basique d'autre part, de la solution résultante présente certaines valeurs prédéterminées. Il est rappelé qu'un ampholyte est un électrolyte possédant à la fois la fonction acide et la fonction basique et présentant par conséquent au moins deux constantes de dissociation, l'une correspondant à la fonction acide, l'autre à la fonction basique.

Ainsi, le brevet précité a proposé une solution aqueuse physiologique pour le lavage des parties du corps humain ou animal ayant été mises en contact avec un acide ou une base, isotonique ou légèrement hypertonique, caractérisée en ce qu'elle comprend, en tant qu'agent(s) actif(s), un ou plusieurs ampholyte(s) choisi(s) de telle sorte que :

-   le pH de la solution soit dans la gamme de 6 à 8,
-   le plus petit des pK acides soit dans la gamme de 6 à 10 et le plus grand des pK basiques soit dans la gamme de 5 à 8, et

- le plus grand pK basique soit inférieur au plus petit pK acide.

Toujours selon le brevet précité, les ampholytes préférés sont le dichlorhydrate d'histidine et le trichlorhydrate d'histidylhistidine, une préférence toute particulière étant donnée au dichlorhydrate d'histidine.

Malheureusement, ce produit est très onéreux et limite de ce fait considérablement le domaine d'utilisation des solutions aqueuses physiologiques le contenant, ce domaine étant jusqu'à présent réservé en pratique aux utilisations dans les sites industriels et dans les laboratoires.

D'autre part, on peut regretter que ce produit ne présente qu'un spectre d'utilisation relativement restreint par rapport aux produits corrosifs et irritants qui peuvent être rencontrés. Il y aurait donc en la matière un intérêt évident à pouvoir disposer d'une solution aqueuse physiologique contenant un produit présentant un spectre d'activité plus large vis-à-vis des agents corrosifs et irritants. Il faut remarquer à ce propos que l'efficacité d'un tel produit ne doit pas simplement être interprétée, ou évaluée, en termes de réaction de neutralisation acide-base mais qu'elle doit surtout être évaluée en fonction de sa capacité à rétablir le plus efficacement et le plus rapidement possible les conditions physiologiques normales préexistant avant l'agression occasionnée par les agents corrosifs et irritants ayant été mis en contact ou ayant été ingérés accidentellement. Ainsi, on recherche à l'heure actuelle une solution qui puisse être efficace non seulement vis-à-vis des acides forts et/ou des bases fortes mais également vis-à-vis des autres produits corrosifs ou irritants.

Les produits proposés et utilisés selon l'art antérieur, tels que le dichlorhydrate d'histidine présentent en outre une efficacité limitée, cette limitation étant essentiellement due au fait que la concentration en produit actif de la solution ne peut dépasser certaines valeurs, celles-ci étant guidées par les pressions osmotiques. On comprendra aisément que l'on ne peut administrer sur telle ou telle partie du corps des solutions physiologiques présentant des pressions osmotiques trop élevées, ce qui limite en pratique la possibilité d'augmenter les concentrations en produit actif des solutions aqueuses physiologiques.

La demande de brevet FR 2 604 900 décrit une solution aqueuse physiologique pour le lavage des parties du corps humain ou animal ayant été mises en contact avec de l'acide fluorhydrique. Cette solution comprend un complexe entre le nitrate d'aluminium et de l'éthylènediamine-tétraacétique tétrasodique.

La demande de brevet japonais JP 3 188 149 décrit une composition de polyacrylate de sodium sous forme de gel dans laquelle le chlorure d'aluminium joue le rôle d'agent de réticulation et l'EDTA peut servir à retarder la formation du gel.

Il y aurait donc un intérêt évident à pouvoir disposer d'un produit ampholyte qui ne soit pas trop onéreux, qui présente un spectre d'utilisation large vis-à-vis des produits corrosifs et irritants, et qui puisse présenter une efficacité notablement plus élevée que ceux déjà connus, à pression osmotique équivalente.

Or, il est du mérite de la Demanderesse d'avoir trouvé et mis au point un tel produit et l'invention a donc pour objet une composition physiologique pour le traitement des brûlures thermiques ou chimiques, cette composition étant caractérisée par le fait qu'elle comprend une quantité efficace d'au moins un complexe formé entre l'ion aluminium $Al^{3+}$, et un ligand éthylènediamine-tétraacétique (EDTA) de formule

$$(CH_2COO^-)_2 \, N\text{-}CH_2\text{-}CH_2\text{-}N(CH_2 \, COO^-)_2.$$

Si l'on dénomme par Y le ligand EDTA, quatre complexes peuvent être formés, répondant à la formule :

$$AlY, \, AlHY, \, Al(OH)Y \text{ ou } Al(OH)_2Y$$

La composition physiologique conforme à l'invention comprend donc une quantité efficace d'au moins l'un de ces complexes et, de façon préférentielle, contient une quantité efficace du complexe Al(OH)Y, ce complexe s'étant en fait révélé le plus efficace en tant qu'ampholyte utilisable pour l'application visée par la présente invention.

Le plus généralement, la composition physiologique selon l'invention comprend une concentration en lesdits complexes de 0,05 mole/l à 1,5 mole/l, de préférence de 0,1 mole/l à 1,0 mole/l et plus préférentiellement encore de 0,1 mole/l à 0,4 mole/l.

Selon une réalisation tout particulièrement avantageuse, la composition conforme à l'invention comprend une concentration en complexe Al(OH)Y de 0,05 mole/l à 1,0 mole/l et de préférence de 0,1 mole/l à 0,6 mole/l et plus préférentiellement encore de 0,1 à 0,4 mole/l.

Bien entendu, lesdits complexes sont, le cas échéant, associés à des cations, dont notamment le $Na^+$.

D'autres ampholytes peuvent également, bien que cela ne s'avère pas forcément indispensable, être présents dans la composition selon l'invention. On peut citer parmi ces ampholytes le citrate disodique, l'alanine, le glutamate disodique, le glycocolle, la lysine, l'alanyl-alanine, le bicarbonate de sodium, l'édétate trisodique (sel trisodique de l'EDTA).

La composition conforme à l'invention peut également contenir d'autres produits communément utilisés dans ce type de compositions tels que du chlorure de sodium et des sucres, notamment du glucose dont la concentration est choisie de manière à ajuster à une valeur donnée l'isotonicité ou l'hypertonicité de la composition selon les applications ou les parties du corps considérées.

La composition physiologique peut également contenir de la glycérine, des glycols, des solvants, notamment des alcools, éventuellement présents pour aider à la solubilisation d'autres composants entrant dans la constitution de ladite composition.

Elle peut en outre contenir des agents de coloration, des agents aromatisants, des agents de goût ou des agents conservateurs ou préservateurs.

Les agents conservateurs et préservateurs sont mis en oeuvre dans les limites autorisées par la réglementation, ces conservateurs et préservateurs pouvant avantageusement être présentés en solution dans le propylène glycol. En tant que conservateur, on peut citer, par exemple la diazolidinylurée, le méthylparaben ou le propylparaben. Ces composés sont généralement compris entre 0,01 % et 0,5 % du poids total de la composition.

La Société demanderesse a en outre découvert que la mise sous forme de "gel" de la composition à base desdits complexes permettait d'obtenir de multiples avantages, dont certains n'étaient a priori pas prévisibles tels que par exemple la réduction des cicatrices keloïdes, la réduction très importante des oedèmes et l'accélération sensible de la cicatrisation, et de procurer aux unités d'urgence ainsi qu'aux particuliers un produit très simple à utiliser, polyvalent et extrêmement efficace.

Au sens de la présente invention, on entend par le terme de gel une composition aqueuse qui présente une viscosité suffisante pour pouvoir persister un temps suffisant sur la peau ou sur les organes brûlés. Cette viscosité est généralement supérieure à 100 Pas (1000 centipoises) et plus généralement comprise entre 200 to 10.000 Pas (2000 et 100 000 cps), de préférence entre 500 et 5 000 Pas (5 000 et 50 000 cps).

La présentation sous forme de gel de la composition pour le traitement interne ou externe des brûlures thermiques ou chimiques permet de combiner de bonnes propriétés thermodynamiques (capacité calorifique, conductivité thermique) et des propriétés osmotiques intéressantes.

Un intérêt évident, aussi bien dans le traitement du brûlé thermique que dans celui du brûlé chimique consiste en effet à avoir une pression osmotique élevée sans être pour autant néfaste. L'osmolalité de la composition conforme à l'invention, qui se présente sous forme de gel, est de 900 à 1100 mosmoles/kg. Cette osmolalité élevée permet dans le cas des brûlures chimiques, de pomper le produit dangereux et, dans le cas d'une brûlure thermique, de résorber l'oedème, permettant ainsi une meilleure nutrition des couches superficielles.

La forme de gel, ou plus généralement cette présentation visqueuse, est obtenue grâce à l'incorporation dans la composition conforme à l'invention d'au moins un épaississant de qualité alimentaire. Les épaississants en question peuvent être par exemple des gommes xanthane, des gommes de guar, des gommes de caroube, des amidons natifs ou modifiés, des carraghénates, de la gomme adragante, de la gomme arabique, des pectines, des éthers de cellulose, dont en particulier les carboxyméthylcelluloses, de l'agar-agar, des alginates.

Ces épaississants peuvent être employés seuls ou en combinaison. De préférence, on utilise, conformément à l'invention, de la gomme xanthane, éventuellement associée à de la gomme de guar ou à de la gomme de caroube.

La quantité d'épaississant que comprend la composition selon l'invention est généralement comprise entre 0,25 et 3 %, de préférence entre 0,5 et 2,5 % et plus préférentiellement encore entre 0,75 % et 2,25 % en poids par rapport au poids total de la composition.

La composition sous forme de gel selon l'invention contient également, avantageusement, de la glycérine, celle-ci aidant à la dispersion de l'épaississant. La glycérine est généralement présente en une quantité de 0,25 % à 5 %, de préférence de 0,5 à 4 % et plus préférentiellement encore de 0,5 à 3 % en poids sur le poids total de la composition.

De préférence, elle contient aussi une huile essentielle, constituée avantageusement par l'huile de tea tree (melalenca alternifolia), dont les remarquables propriétés germicides, bactéricides et antifongiques sont bien connues. La concentration en huile essentielle de la composition peut être établie entre 0,10 % et 2 %, de préférence entre 0,15 % et 1,5 % et plus préférentiellement encore entre 0,2 % et 1 % en poids sur le poids total de la composition.

Un agent tensio-actif, ou agent mouillant, peut être ajouté à la composition sous forme de gel conforme à l'invention. Lors de la fabrication du gel, il aide à la dispersion de l'épaississant, et dans le produit final il contribue à l'adhérence du gel à la peau et aux vêtements du brûlé. Cet agent tensio-actif est généralement ajouté en une quantité comprise entre 0,010 % et 0,75 %, et de préférence entre 0,050 % et 0,50 % en poids sur le poids total de la composition. Il est avantageusement constitué par de l'octoxynol ou du capryl caprilyl glucoside.

La composition sous forme de gel selon l'invention comprenant au moins un épaississant de qualité alimentaire et, avantageusement, de la glycérine, une huile essentielle ainsi qu'un agent tensio-actif est de préférence présentée dans un récipient sous pression, afin de faciliter son application.

La composition sous forme de gel conforme à l'invention permet d'obtenir une bien meilleure efficacité que les solutions pour le traitement des brûlures thermiques ou chimiques de l'art antérieur et permet également aux équipes d'urgence, ainsi qu'éventuellement aux particuliers, de n'avoir recours qu'à un seul produit, quel que soit le type de brûlure.

De préférence, la composition sous forme de gel conforme à l'invention est présentée dans un récipient sous pression, permettant une application très aisée.

La composition selon l'invention présente avantageusement un pH compris entre 6 et 9.

A ce titre, on signale que, de façon avantageuse, on peut stabiliser le pH à l'aide d'acide borique et/ou de glycocolle. L'acide borique et le glycocolle présentent en outre l'avantage de renforcer l'action anti-base.

La composition conforme à l'invention, grâce à son caractère amphotère et chélateur, stoppe la progression des produits chimiques aspergés ou ingérés accidentellement, mais permet également de réduire l'acidose résultant des brûlures thermiques.

L'invention a également pour objet un procédé de fabrication de la composition physiologique conforme à l'invention, ce procédé étant caractérisé par le fait que l'on oxyde de l'aluminium métal dans une solution alcaline forte, préférentiellement une solution de soude, que l'on complexe l'ion aluminium en solution basique ainsi obtenu par de l'EDTA acide, que l'on amène ensuite la solution obtenue à la concentration désirée, avec éventuellement introduction des additifs, tampons et solvants voulus.

L'aluminium, bien qu'étant un métal très réducteur ($E_o$ = - 1,66 v) se recouvre en solution aqueuse d'une pellicule protectrice d'oxyde entre pH 5 et 11. Lorsque l'on soumet l'aluminium à l'action de solutions alcalines, la couche d'oxyde se dissout et il se forme des aluminates de formule Na [Al(OH)$_4$].

L'aluminium mis à nu réagit également en formant des aluminates.

L'équation globale décrivant le processus de dissolution de l'aluminium dans une solution aqueuse d'alcali est donc la suivante :

$$2Al + 2NaOH + 6H_2O \rightarrow 2Na\,[Al(OH)_4]$$

La réaction de complexation de l'EDTA acide est ensuite réalisée sur la solution alcaline d'aluminates ainsi obtenue. Cette réaction de complexation est relativement délicate à réaliser car l'aluminium se complexe habituellement avec l'EDTA tétrasodique. Ici, en l'occurrence, il faut réaliser dans la même opération la dissolution de l'EDTA acide et la complexation avec l'ion aluminium.

Afin que cette complexation puisse se réaliser au mieux, l'EDTA acide est préférentiellement ajouté rapidement et en une seule fois dans la solution d'aluminates, à température élevée, de préférence proche de la température d'ébullition, et sous agitation.

Si l'EDTA est ajouté peu à peu, l'ion aluminium se trouve en excès au cours de la réaction et il y a formation d'hydroxyde. De même, si on procède à température peu élevée, l'EDTA ne se dissout pas correctement et il se forme alors un dépôt au fond du réacteur. Dans les deux cas, les rendements de formation des complexes recherchés sont donc moins bons.

L'équation de formation du complexe préféré s'écrit globalement comme suit (Y représentant toujours le ligand EDTA) :

$$Na[Al(OH)_4] + NaOH + H_4Y \rightarrow Na_2\,Y\,AlOH + 4H_2O$$

De préférence, la température de réaction au moment de l'addition de l'EDTA acide est supérieure à 70°C et de préférence supérieure à 85°C environ.

Le produit réactionnel obtenu en fin de réaction comprend les complexes AlY, AlYH, AlYOH, Al(OH)$_2$Y et essentiellement le complexe AlYOH préférentiellement recherché.

Ce produit réactionnel peut être vendu tel quel, éventuellement après évaporation ou mise sous forme de poudre, par exemple par atomisation, et forme alors un concentré aqueux ou un concentré en poudre, utilisable pour la préparation des solutions physiologiques finales.

Celles-ci sont préparées par dilution desdits concentrés avec de l'eau à usage physiologique jusqu'à la concentration en complexes actifs désirée, avec éventuellement addition d'autres ampholytes, agents tampons ou correcteurs de pH, conservateurs, colorants, co-solvants ou autres additifs. Cette solution est ensuite stérilisée. Lorsque l'on présente la composition conforme à l'invention sous forme de gel, on ajoute sous agitation à une solution comprenant au moins un complexe AlY, AlHY, Al(OH)Y ou Al(OH)$_2$Y une quantité suffisante d'au moins un épaississant alimentaire, tels que ceux décrits précédemment, ainsi qu'éventuellement, de la glycérine, une huile essentielle, constituée préférentiellement par l'huile de tea tree ou un agent tensioactif ; puis on conditionne la composition ainsi obtenue, de préférence dans un emballage sous pression, et l'on procède ensuite à une stérilisation.

De préférence, la préparation desdites compositions s'effectue en salle blanche.

La composition selon l'invention peut être utilisée pour un usage externe, pour le refroidissement des brûlures thermiques, pour l'arrêt et le lavage des brûlures chimiques ou pour la réduction des oedèmes et la protection des brûlures. Elle peut également être utilisée pour un usage interne, dans le cas par exemple de brûlures chimiques gastriques, afin d'arrêter la progression du produit chimique et afin de réaliser un pansement oesophagien.

Des exemples de compositions conformes à l'invention sont donnés ci-après, à titre purement illustratif.

EXEMPLE 1 : Préparation d'un concentré de complexes conforme à l'invention

Dans un ballon réactionnel muni d'un extracteur de gaz, on introduit la quantité d'eau nécessaire puis on fait dissoudre la quantité de soude en pastilles voulue avant d'ajouter l'aluminium. Quand l'aluminium est dissous, on chauffe la cuve à une température de 90°C et on ajoute l'EDTA acide en une fois en agitant fortement jusqu'à dissolution du produit.

Les quantités utilisées ramenées en gramme par litre sont les suivantes :

- soude : 28 g/l,
- aluminium : 8,5 g/l,
- EDTA acide : 94 g/l.

Le concentré ainsi obtenu peut être utilisé pour la préparation de solution physiologique conforme à l'invention.

EXEMPLE 2 :

Une solution aqueuse physiologique est ainsi préparée à partir dudit concentré, cette solution aqueuse comprenant :

- sel disodique de AlOHY (poids moléculaire de 378,20) : 98,3 g/l, soit 0,26 mole/l,
- sel monosodique de AlY (poids moléculaire de 418,20) : 25 g/l, soit 0,06 mole/l,
- acide orthoborique (poids moléculaire de 61,8) : 21 g/l, soit 0,34 mole/l,
- chlorure de sodium (poids moléculaire de 58,44) : 12,8 g/l, soit 0,22 mole/l,
- eau déminéralisée, qsp : 1 litre.

EXEMPLE 3 :

Une autre solution aqueuse conforme à l'invention est préparée avec la composition suivante :

- sel disodique de l'ion AlYOH : 35,3 g,
- glycocolle : 7,0 g,
- chlorure de sodium : 16,3 g,
- méthyl hydroxy-4 benzoate sodé : 0,5 g,
- eau déminéralisée, qsp : 1000 ml.

EXEMPLE 4 :

Une autre solution aqueuse physiologique conforme à l'invention présente la composition suivante :

- sel disodique de AlYOH : 106,0 g,
- glycocolle : 21,0 g,
- chlorure de sodium : 12,8 g,
- méthyl hydroxy-4 benzoate sodé : 0,5 g,

- eau déminéralisée, qsp : 1000 ml.

Conformément à la législation en vigueur pour les produits cosmétiques et d'hygiène corporelle, les tests d'innocuité ont été effectués sur les produits, notamment sur la composition de l'exemple 2 et ont montré que :

- le produit est considéré comme non irritant par voie cutanée chez le lapin ; l'indice d'irritation primaire cutanée est ainsi de 0,5.
- le produit est considéré comme non irritant par voie oculaire chez le lapin.

Aucune réaction oculaire n'est observée chez les animaux, l'indice d'irritation oculaire est de 0,0.

- la DL 0 du produit administré par voie orale chez le rat est supérieure ou égale à 5000 mg/kg, dose à laquelle aucun signe de toxicité n'est observé.

EXEMPLE 5 : COMPOSITION POUR USAGE EXTERNE

La formulation est la suivante :

- complexe ampholyte : acide éthylènediamine tétraacétique, sel disodé (sel disodique de Al(OH)Y, de poids moléculaire de 378,20)    3,60 g
- acide orthoborique    0, 55 g
- chlorure de sodium    1,60 g
- gomme xanthane de marque KELTROL, commercialisée par la Société KELCO    1,25 g
- glycérine    1,00 g
- tensio-actif non ionique de marque TRITON CG-110, commercialisée par la Société UNION CARBIDE    0,10 g
- Germaben II
  propylène glycol : 56 %
  diazolidinylurée : 30 %
  méthylparaben : 11 %
  propylparaben : 3 %    1,00 g
- huile essentielle de tea tree    0,50 g
- eau déminéralisée qsp    100 ml

EXEMPLE 6 : FORMULATION POUR USAGE INTERNE

La formulation comprend les produits suivants :

- complexe ampholyte :
  acide éthylènediaminetétraacétique, sel disodé, complexe d'aluminium hydroxyde (sel disodique de Al(OH)Y de poids moléculaire 378,20)    9,60 g
  acide éthylène diaminetétraacétique, sel trisodé, complexe d'aluminium (sel trisodique de $Al(OH)_2Y$, de poids moléculaire 418,20)    2,45 g

- acide orthoborique 2,05 g
- gomme xanthane de marque KELTROL, commercialisée par la Société KELCO 1,25 g
- glycérine 1,00 g
- tensio-actif non ionique de marque TRITON CG-110, commercialisée par la Société UNION CARBIDE 0,10 g
- eau déminéralisée 100 ml

L'osmolalité desdites compositions se situe entre 900 et 1100 mosmoles/kg. Elle est donc plus élevée que l'osmolalité d'une solution correspondante comprenant les mêmes complexes ampholytes, cette osmolalité étant alors d'environ 850 mosmoles/kg. Elle est d'autre part beaucoup plus élevée que l'osmolalité d'une solution de 9 % de chlorure de sodium, cette osmolalité s'établissant aux environs de 290 mosmoles/kg.

Les deux formulations ci-dessus ont été évaluées du point de vue toxicologique.

Elles sont considérées comme non irritantes sur la peau d'après un test d'irritation cutanée sur le lapin.

Elles sont également non irritantes sur les yeux d'après les résultats d'un test d'irritation oculaire chez le lapin.

La DL50 sur peau intacte chez le rat est supérieure à 2000 mg/kg, et la composition est donc considérée comme non toxique sur la peau.

La DL 50 orale sur le rat est quant à elle supérieure à 2000 mg/kg, dose à laquelle aucun signe de toxicité ne peut être observé.

Ces données montrent la parfaite innocuité de la composition conforme à l'invention.

On dispose ainsi, grâce à l'invention, d'une composition efficace, polyvalente, simple d'utilisation et d'une parfaite innocuité pour le traitement interne ou externe des brûlures chimiques ou thermiques.

**Revendications**

1. Composition physiologique pour le traitement des brûlures thermiques ou chimiques, **caractérisée par le fait qu'**elle comprend une quantité efficace du complexe Al(OH)Y formé entre l'ion Aluminium $Al^{3+}$ et un ligand éthylènediamine-tétraacétique (ligand Y).

2. Composition physiologique selon la revendication 1, **caractérisée par le fait qu'**elle comprend une concentration en ledit complexe Al(OH)Y de 0,05 mole/l à 1,5 mole/l, de préférence de 0,1 mole/l à 1,0 mole/l et plus préférentiellement encore de 0,1 mole à 0,4 mole/l.

3. Composition physiologique selon l'une quelconque des revendications 1 à 2, **caractérisée par le fait qu'**elle se présente sous la forme d'une solution aqueuse de pH compris entre 6 et 9.

4. Composition physiologique selon l'une quelconque des revendications 1 à 3, **caractérisée par le fait qu'**elle contient du chlorure de sodium et des sucres afin d'ajuster sa tonicité et éventuellement d'autres ampholytes, de la glycérine, des glycols, des solvants.

5. Composition physiologique selon l'une quelconque des revendications 1 à 4, **caractérisée par le fait qu'**elle se présente sous la forme d'un gel.

6. Composition physiologique selon la revendication 5, **caractérisée par le fait qu'**elle comprend au moins un épaississant de qualité alimentaire, en une quantité de 0,25 à 3 %, de préférence de 0,5 % à 2,5 % et plus préférentiellement encore de 0,75 % à 2,25 % en poids sur le poids total de la composition.

7. Composition physiologique selon l'une ou l'autre des revendications 5 et 6, **caractérisée par le fait qu'**elle comprend de la glycérine, en une quantité de 0,25 % à 5 %, de préférence de 0,5 % à 4 % et plus préférentiellement encore de 0,5 % à 3 % en poids sur le poids total de la composition.

8. Composition physiologique selon l'une quelconque des revendications 5 à 7, **caractérisée par le fait qu'**elle comprend une huile essentielle, en une quantité comprise entre 0,10 % et 2 %, de préférence entre 0,15 % et 1,5 % et plus préférentiellement encore entre 0,2 % et 1 % en poids sur le poids total de la composition.

9. Composition physiologique selon l'une quelconque des revendications 5 à 8, **caractérisée par le fait qu'**elle comprend un agent tensio-actif, de préférence en une quantité comprise entre 0,010 et 0,75 %, et plus préférentiellement encore en une quantité comprise entre 0,050 % et 0,50 % en poids sur le poids total de la composition.

10. Procédé de préparation d'une composition physiologique selon l'une quelconque des revendications 1 à 9, **caractérisé par le fait que** l'on oxyde de l'aluminium métal dans une solution alcaline forte, préférentiellement une solution de soude, que l'on complexe l'ion aluminium en solution basique ainsi obtenu par de l'EDTA acide, que l'on amène ensuite la solution obtenue à la concentration désirée, avec éventuellement introduction des additifs, tampons et solvants voulus.

11. Procédé selon la revendication 10, **caractérisé par le fait que** l'addition de l'EDTA acide est réalisée à température élevée, de préférence supérieure à 70°C, et plus préférentiellement encore supérieure à 85°C.

12. Procédé selon l'une ou l'autre des revendications 10 ou 11, **caractérisé par le fait que** l'addition de l'EDTA acide est réalisée rapidement.

13. Procédé de préparation d'une composition selon l'une quelconque des revendications 5 à 9, **caractérisé par le fait qu'**on ajoute à une solution comprenant du complexe Al(OH)Y sous agitation, une quantité suffisante d'au moins un épaississant alimentaire, et éventuellement de la glycérine, une huile essentielle, un agent tensio-actif, que l'on conditionne ensuite la composition ainsi obtenue, de préférence dans un emballage sous pression, et que l'on effectue ensuite une stérilisation.

14. Concentré réactionnel utile pour la préparation d'une composition physiologique selon l'une quelconque des revendications 1 à 9, comprenant du complexe Al(OH)Y.

15. Utilisation de la composition selon l'une quelconque des revendications 1 à 9 pour l'obtention d'un médicament destiné au traitement des brûlures internes ou externes, à l'exception du traitement des brûlures par l'acide fluorhydrique..

16. Utilisation d'une composition physiologique comprenant une quantité efficace d'un complexe AlY (OH) formé entre l'ion Aluminium $Al^{3+}$ et un ligand éthylènediamine-tétraacétique (ligand Y) pour l'obtention d'un médicament destiné au traitement des brûlures internes ou externes à l'exception du traitement des brûlures par l'acide fluorhydrique.

**Patentansprüche**

1. Physiologische Zusammensetzung zur Behandlung von thermischen oder chemischen Verbrennungen, **dadurch gekennzeichnet, dass** sie eine wirksame Menge des Komplexes Al(OH)Y umfasst, der zwischen dem Aluminiumion $Al^{3+}$ und einem Ethylendiamintetraacetatliganden (Ligand Y) gebildet ist.

2. Physiologische Zusammensetzung gemäß Anspruch 1, **dadurch gekennzeichnet, dass** sie eine Konzentration des Al(OH)Y-Komplexes von 0,05 Mol/$\ell$ bis 1,5 Mol/$\ell$, bevorzugt 0,1 Mol/$\ell$ bis 1,0 Mol/$\ell$ und noch bevorzugter 0,1 Mol bis 0,4 Mol/$\ell$ umfasst.

3. Physiologische Zusammensetzung gemäß einem der Ansprüche 1 bis 2, **dadurch gekennzeichnet,**

**dass** sie in Form einer wässrigen Lösung mit einem pH zwischen 6 und 9 vorliegt.

4. Physiologische Zusammensetzung gemäß einem der Ansprüche 1 bis 3, **dadurch gekennzeichnet, dass** sie Natriumchlorid und Zucker enthält, um die Tonizität einzustellen, und gegebenenfalls weitere Ampholyte, Glycerin, Glykole, Lösungsmittel.

5. Physiologische Zusammensetzung gemäß einem der Ansprüche 1 bis 4, **dadurch gekennzeichnet, dass** sie in Form eines Gels vorliegt.

6. Physiologische Zusammensetzung gemäß Anspruch 5, **dadurch gekennzeichnet, dass** sie mindestens ein Verdickungsmittel von Nahrungsmittelqualität in einer Menge von 0,25% bis 3%, bevorzugt 0,5% bis 2,5%, und noch bevorzugter 0,75% bis 2,25 Gew.% umfasst, bezogen auf das Gesamtgewicht der Zusammensetzung.

7. Physiologische Zusammensetzung gemäß dem einen oder dem anderen der Ansprüche 5 und 6, **dadurch gekennzeichnet, dass** sie Glycerin in einer Menge von 0,25% bis 5%, bevorzugt 0,5% bis 4%, und noch bevorzugter 0,5% bis 3 Gew.% des Gesamtgewichts der Zusammensetzung umfasst.

8. Physiologische Zusammensetzung gemäß einem der Ansprüche 5 bis 7, **dadurch gekennzeichnet, dass** sie ein essentielles Öl in einer Menge zwischen 0,10% und 2%, bevorzugt zwischen 0,15% und 1,5% und noch bevorzugter zwischen 0,2% und 1 Gew.%, bezogen auf das Gesamtgewicht der Zusammensetzung umfasst.

9. Physiologische Zusammensetzung gemäß einem der Ansprüche 5 bis 8, **dadurch gekennzeichnet, dass** sie ein tensioaktives Mittel umfasst, bevorzugt in einer Menge zwischen 0,010 und 0,75%, und noch bevorzugter in einer Menge zwischen 0,050% und 0,50% Gew.%, bezogen auf das Gesamtgewicht der Zusammensetzung.

10. Verfahren zur Herstellung einer physiologischen Zusammensetzung gemäß einem der Ansprüche 1 bis 9, **dadurch gekennzeichnet, dass** man Aluminiummetall in einer stark alkalischen Lösung oxidiert, bevorzugt in einer Lösung von Natronlauge, dass man das so erhaltene Aluminiumion in einer basischen Lösung mit EDTA-Säure komplexiert, dass man die erhaltene Lösung auf die gewünschte Konzentration einstellt, gegebenenfalls mit der Einschleusung von Additiven, Puffern und gewünschten Lösungsmitteln.

11. Verfahren gemäß Anspruch 10, **dadurch gekennzeichnet, dass** die Zugabe von EDTA-Säure bei einer erhöhten Temperatur durchgeführt wird, bevorzugt über 70°C, und noch bevorzugter über 85°C.

12. Verfahren gemäß dem einen oder dem anderen der Ansprüche 10 oder 11, **dadurch gekennzeichnet, dass** die Zugabe von EDTA-Säure schnell durchgeführt wird.

13. Verfahren zur Herstellung einer Zusammensetzung gemäß einem der Ansprüche 5 bis 9, **dadurch gekennzeichnet, dass** man zu einer Lösung, die Al (OH)Y-Komplex umfasst, unter Rühren eine ausreichende Menge von mindestens einem Lebensmittelverdickungsmittel und gegebenenfalls Glycerin, ein essentielles Öl, ein tensioaktives Mittel, hinzugibt, dass man die so erhaltene Zusammensetzung bevorzugt unter Druck in eine Verpackung einfüllt, und dass man anschließend eine Sterilisierung durchführt.

14. Wirkstoffkonzentrat, das zur Herstellung einer physiologischen Zusammensetzung gemäß einem der Ansprüche 1 bis 9 nützlich ist, umfassend einen Al (OH)Y-Komplex.

15. Verwendung der Zusammensetzung gemäß einem der Ansprüche 1 bis 9 zum Erhalt eines Medikamentes, das auf die Behandlung von inneren oder äußeren Verbrennungen ausgerichtet ist, mit der Ausnahme einer Behandlung von Verbrennungen durch Flusssäure.

16. Verwendung einer physiologischen Zusammensetzung umfassend eine wirksame Menge eines AlY (OH)-Komplexes, der zwischen dem Aluminiumion $Al^{3+}$ und einem Ethylendiamintetraacetatliganden (Ligand Y) zum Erhalt eines Medikamentes, das auf eine Behandlung von internen oder externen Verbrennungen ausgerichtet ist, mit der Ausnahme einer Behandlung von Verbrennungen durch Flusssäure.

**Claims**

1. Physiological composition for the treatment of thermal or chemical burns **characterized by** the fact that it includes an efficient amount of the Al(OH)Y complex formed between the aluminium ion $Al^{3+}$ and an ethylenediamine-tetraacetic ligand (ligand Y).

2. Physiological composition according to the claim 1, **characterized by** the fact that it includes a concentration of said Al(OH)Y complex of 0.05 mole/l to 1.5 mole/l, preferably from 0.1 mole/l to 1.0 mole/l and most preferably still from 0.1 mole/l to 0.4 mole/l.

3. Physiological composition according to any one of the claims 1 to 2, **characterized by** the fact that it is made available in the form of an aqueous solution of pH comprised between 6 and 9.

4. Physiological composition according to any one of the claims 1 to 3, **characterized by** the fact that it contains sodium chloride and sugars in order to adjust its tonicity and as the case may be, other ampholytes, glycerol, glycols, solvents.

5. Physiological composition according to any one of the claims 1 to 4, **characterized by** the fact that it is made available in the form of a gel.

6. Physiological composition according to the claim 5, **characterized by** the fact that it includes at least one thickener of a nutritional quality, in an amount from 0.25 to 3, preferably from 0.5 to 2.5 and most preferably still from 0.75 to 2.25 weight % related to the total weight of the composition.

7. Physiological composition according to any of the claims 5 and 6, **characterized by** the fact that it contains glycerol in an amount from 0.25 to 5, preferably from 0.5 to 4 and most preferably still from 0.5 to 3 weight % related to the total weight of the composition.

8. Physiological composition according to any one of the claims 5 to 7, **characterized by** the fact that it includes an essential oil, in an amount comprised between 0.10 and 2, preferably between 0.15 and 1.5 and most preferably still between 0.2 and 1 weight % related to the total weight of the composition.

9. Physiological composition according to any one of the claims 5 to 8, **characterized by** the fact that it comprises a surfactant, preferably in an amount comprised between 0.010 and 0.75, and most preferably still in an amount comprised between 0.050 and 0.50 weight % related to the total weight of the composition.

10. Process for the preparation of a physiological composition according to any one of the claims 1 to 9, **characterized by** the fact that metal aluminium is oxided in a strong alkaline solution preferably a soda solution, the aluminium ion is complexed in basic solution thus obtained by acid EDTA, and the solution obtained is then brought to the desired concentration with the possible introduction of additives, buffers and solvents as required.

11. Process according to claim 10, **characterized by** the fact that the addition of acid EDTA is performed at high temperature, preferably higher than 70°C, and most preferably higher than 85°C.

12. Process according to any one of the claim 10 or 11, **characterized by** the fact that the addition of acid EDTA is performed rapidly.

13. Process for the preparation of a composition according to any one of the claims 5 to 9, **characterized by** the fact that there is added to a solution comprising Al(OH)Y complex, under stirring, a sufficient amount of at least one nutritional thickener and as the case may be glycerol, an essential oil, a surfactant, and the composition thus obtained is subsequently conditioned, preferably in a pressurized packing and a sterilization is then carried out.

14. Reaction concentrate useful for the preparation of a physiological composition according to any one of the claims 1 to 9, comprising Al(OH)Y complex.

15. Use of the composition according to any one of the claims 1 to 9 for the treatment of internal or external burns, except for the treatment of fluorhydric acid burns.

16. Use of a physiological composition comprising an efficient amount of a AlY(OH) complex formed between the aluminium ion $Al^{3+}$ and ethylenediaminetetraacetic ligand (ligand Y), for the manufacture of a medicament for the treatment of internal or external burns, except for the treatment of fluorhydric acid burns.